# EUROPEAN PATENT SPECIFICATION

(11) **EP 1 088 529 B1**
(45) Date of publication and mention of the grant of the patent: **01.06.2005**
(21) Application number: 00121510.2
(22) Date of filing: 29.09.2000
(51) Int. Cl.: A61F 2/24

(54) **A device for cardiac valve replacement or repair operations**
Vorrichtung für Herzklappenersatz oder Reparaturoperationen
Dispositif pour remplacement et réperation de valves cardiaques

(30) Priority: 30.09.1999 IT TO990840
(43) Date of publication of application: 04.04.2001
(73) Proprietor: SORIN BIOMEDICA CARDIO S.R.L., 13040 Saluggia (Vercelli) (IT)
(72) Inventor: Arru, Pietro, 10020 Marentino (Torino) (IT); Vallana, Franco, 10123 Torino (IT); Bona, Gioachino, 10128 Torino (IT); Stacchino, Carla, 10132 Torino (IT)
(74) Representative: Gerbino, Angelo

(56) References cited:
- EP-A- 0 896 813
- WO-A-98/57599
- DE-A- 4 022 460
- US-A- 5 957 949

## Description

The present invention relates to a device for cardiac valve replacement or repair operations.

In more detail, repair operations are undertaken by introduction into a valve site of an annuloplasty ring so as to renew the original geometry of the annulus and permit reinstatement of the correct function of the natural valve.

Implant devices used for repair operations are constituted by a closed or partially open annular structure reproducing the geometry of the inlet edge of the mitral valve. They comprise a more or less flexible inner core and an outer cladding of polyester or polytetrafluoroethylene (PTFE) fabric which allows surgical suture.

In the case of replacement operations, on the other hand, the entire natural valve is removed and replaced with a cardiac valve prosthesis.

As is known to those skilled in the art cardiac valve prostheses can be classified into two fundamental categories: mechanical prostheses and biological prostheses.

Mechanical valve prostheses are usually constituted by an annular frame of generally rigid material intended to be connected by a ring of suture to the annulus of the natural valve which is removed. In the annular frame are mounted one or more valve obturator members which move under the action of the blood. Biological valves are usually formed starting from sheets of suitably treated biological tissue of animal origin.

Whatever the nature of the operation performed and, in the case of replacement operations, the type of prosthesis used, the implantation technique essentially used until now was a surgical technique by sternotomy. Although by now well consolidated the conventional surgery is, however, very traumatic and not free from complications for the patient, above all in the presence of concomitant risk factors (for example advanced age, other pathologies and the like).

For this reason, and also taking into consideration the significant development which has occurred in the last years in adjacent fields with various operating techniques involving percutaneous access, such as angioplasty of coronary vessels, mapping of the walls of the cardiac cavity with a view to ablation of arrhythmia etc., there is an increasing interest in producing devices such as rings for annuloplasty and cardiac valve prostheses, capable of being introduced with low-invasive surgery techniques. Such techniques, as far the cardiac sector is concerned, include both surgical access via minithoracotomy with direct or assisted vision in the operating field, and endoluminal percutaneous access.

There is therefore a need to provide rings for annuloplasty and cardiac valve prostheses which lend themselves to application with the above mentioned low-invasive surgery techniques in order to be then unfolded in the valve site of interest and fixed in position.

This choice presupposes an almost obligatory recourse to devices which have characteristics of flexibility so that they can be folded into compact dimensions during introduction in order subsequently to become unfolded in the implant position. Biological valves, for example of the types described in European Patent EP-B-0 155 245 in which the biological tissue used to form the valve membrane is a different biological tissue from the valve tissue (for example bovine pericardium) has shown itself to be particularly suitable in this respect, at least potentially.

In particular, it is known (see for example document EP-A-0 515 324) that biological valves of the above-specified type lend themselves to being produced in the form of valves free from rigid or substantially rigid annular support frames.

DE-A-40 22 460 discloses a device for cardiac valve replacement, which is provided with a support formation which can adopt a first, generally contracted shape and a second, generally unfolded shape. The preamble of claim 1 which follows reflects the state of the art corresponding to such prior art document.

US-A-5 957 949 discloses a percutaneously implanted valve stent comprising a support formation made of shape memory alloy.

WO-A-9 857 599 refers to prosthetic venous valves having fixing means made of shape memory alloy, which alloy different from the alloy forming the value support.

The object of the present invention is that of providing a device for cardiac valve replacement or repair operations which can be unfolded in situ so as to allow them to be introduced by low-invasive surgery techniques.

According to the present invention this object is achieved by a device having the characteristics specifically set out in the following Claims.

The invention will now be described purely by way of non-limitative example, with reference to the annexed drawings, in which:
Figure 1 illustrates in plan a first embodiment of a device according to the invention;
Figure 2 is a section view taken on the line II-II of figure 1;
Figure 3 illustrates in plan another embodiment of the device according to the invention; and
Figure 4 is a perspective illustration of a further embodiment of a device according to the invention.

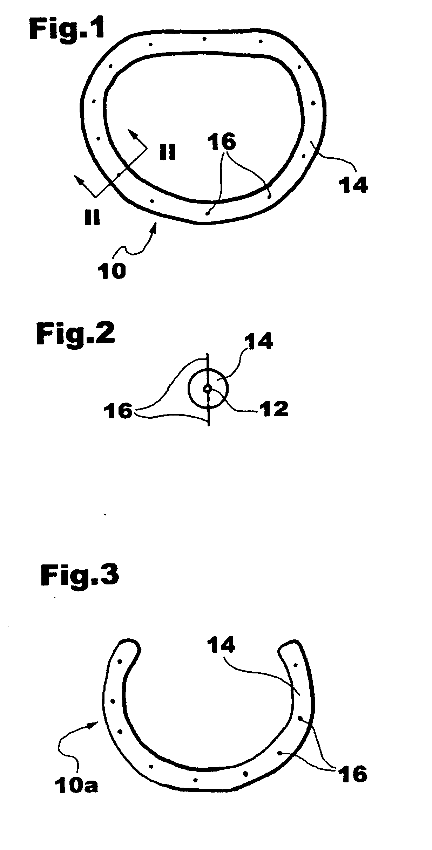

In Figures 1 and 2 the reference 10 indicates a ring for annuloplasty comprising, as a central core, an annular support formation 12 of a shape-memory material such as, for example, the metal material known as nitinol (an alloy based on Ti-Ni).

The support formation 12 is embedded in an annular casing 14 of biocompatible material such as, for example, polyester or PTFE textile.

The support formation 12 is moreover provided with a plurality of spaced projections 16 which extend transversely with respect to the general plane of the ring 10 and are formed of a shape-memory material, preferably having different properties from those of the material used for the support formation 12.

The ring 10 can be introduced at a valve site, the original geometry of the annulus of which needs reinstatement, using low-invasive surgery techniques by means of suitable manipulating instruments.

In both cases the support formation 12 and therefore the ring 10 as a whole is first caused to assume a contracted form of reduced dimensions, which considerably facilitates and shortens the introduction operation.

Then, having reached the implant valve site, the ring 10 is unfolded, for example by heating it to a temperature greater than that of the body during operating conditions, preferably of the order of 50°C so that the support formation 12 reassumes the substantially circular shape illustrated in Figure 1. The methods by which this heating is achieved are conventional and some of these will be described in more detail hereinafter with reference to another embodiment of the invention.

Subsequently, by further heating to a higher temperature, for example of the order of 60°C, the projections 16 pass from the substantially rectilinear shape illustrated in the drawing to a hook shape projecting outwardly from the ring 10, which thus grip onto the natural tissue of the implant site and become securely anchored to it.

In this way the expansion of the ring 10 and its anchorage system are achieved independently from one another. This makes it possible for the surgeon to effect first of all optimum orientation of the ring 10 in relation to the surrounding anatomical structures upon expansion, and subsequently to anchor it to the implant site by means of expansion of the projections 14. The original geometry of the annulus is thus reinstated and the associated valve can again function in a correct manner.

Figure 3 illustrates a variant embodiment 10a of a ring for annuloplasty which differs from that described with reference to the preceding Figures only by the fact that it has an open rather than a closed loop structure, the method of introduction and unfolding in situ remaining unchanged.

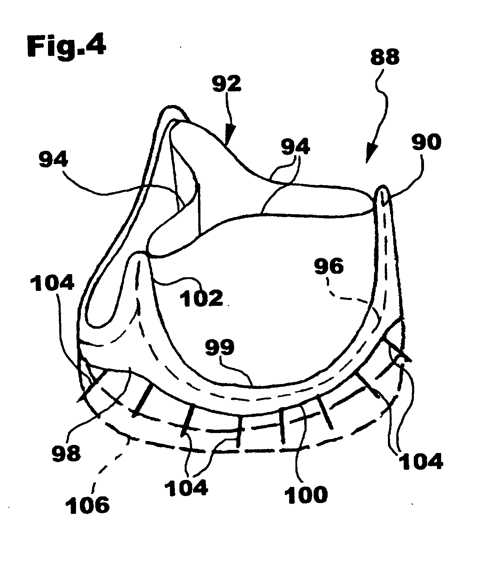

Figure 4 illustrates a device according to the invention formed as a cardiac valve prosthesis, generally indicated with the reference 88.

As already previously indicated, this type of prosthesis constitutes a development of the arrangement described in prior art documents EP-B-0 155 245 and EP-A-0 515 324.

For a complete description of the manufacturing details of the prosthesis (also as far as the processes and apparatus usable to produce the shaping of the component parts are concerned) reference can in general be made to the description in the two preceding documents: solely all the elements characteristic of the arrangement according to the invention will be described explicitly in the present description. Other elements not specifically illustrated must, however, be considered to be known to the man skilled in the art (for example through the knowledge of the two documents to which reference has been made several times) and in any event are not, per se, relevant for the purposes of understanding the invention.

The prosthesis 88 is essentially constituted by a sleeve comprising two sheets 90 and 92 of biological material such as, for example, bovine pericardium subject to a fixation process in glutaraldehyde.

The first sheet 90 is intended to constitute the outer portion of the valve sleeve of the prosthesis 88. The other sheet 92, constitutes the inner or functional part of the valve sleeve (that is to say, in practice, the valve lips) has been preliminarily subject to a shaping process. This process is intended to give the sheet 92 three shaped portions 94 of semi-circular or crescent shape with arcuate edges and with a generally cup or claw like configuration in such a way that, when the valve sleeve is closed in a tube, the flaps 94 project in a convergent manner towards the internal orifice of the prosthesis 88. A detailed description of a process and apparatus usable for performing this shaping operation is provided in European Patent EP-B-0 133 420.

The two sheets 90 and 92 are then connected together by a line of suture stitching (for example of spun polyester thread covered in biocompatible carbonaceous material) which extends closely adjacent to the outer outline of the crescent portions forming the valve membranes 94 and therefore has a festoon shape.

More precisely, the two sheets 90 and 92 connected together by the festoon line of stitching 96, are closed in a tube and connected together at their facing ends by means of a further line of stitching 98. This is done in such a way as to give rise to a structure in which the three valve membranes 94 can, alternatively, move away from one another so as to allow the flow of blood in one direction (from below upwardly with reference to the drawing) and to move together again against one another under the act of the blood pressure so as to prevent the flow of blood in the opposite direction. All this according to criteria widely known in the art, which do not require to be further explained here.

Preferably, the stitch line 98 is located at one of the so-called commissures that is to say in the region of maximum axial extent of the sheets 90 and 92.

As well as this, the edge 99 of the outer sheet 90 intended to be situated down stream in the implant position of the valve (that is to say the edge intended to face towards the output side of the valve - orientated upwardly when the prosthesis is located as illustrated in Figure 4) is cut off in close proximity to the festoon line of stitching 96, and therefore in close proximity to the arcuate edges of the valve flaps 94.

It will be appreciated that this choice (the distance between edge 99 and the stitch line of the order, for example, of 2mm) is closer to the arrangement described in document EP-B-0 155 245 (which describes a valve provided with a reinforcement or frame), than to the arrangement described in document EP-A-0 515 324 which relates to a valve without reinforcement or frame.

However, in the prosthesis 88 according to the invention it is preferential that the outer sheet has a very reduced axial dimension at its margin on the output side for the purposes of production of a valve prosthesis intended to be introduced with low-invasive surgery techniques, above all when the operation is performed for the purpose of replacing an aortic valve so as not to obstruct the coronary ostia.

The prosthesis 88 can to some extent be comparable to a valve provided with a reinforcement frame due to the presence, preferably at the base perimeter of the prosthesis itself, of an annular support formation 100 (preferably continuous, although a discontinuous structure formed from several arcuate segments is possible) made of a shape memory material such as, for example, the above mentioned nitinol.

The connection of the annular formation 100 to the sheets of biological material 90, 92 is usually achieved by stitching, for example in a manner substantially similar to that described in document EP-B-0 155 245 for the connection of the base region of the reinforcement frame or stent to the sheets of biological or other material again in a manner similar to that adopted for the connection of the same sheet together in the base region (ingress or inflow side of the prosthesis) in the arrangement described in document EP-A-0 515 324.

It will be appreciated that, in general, the support formation 100 is preferably located in direct connection, or at least in close proximity, to the base region (inflow side) of both sheets of biological material.

As will be seen better hereinafter, the formation 100 has in fact the purpose of ensuring the unfolding of the prosthesis in the implant position: it is therefore important that both the outer sleeve 90 and the inner sleeve 92 which carries the valve membranes 94 are accurately unfolded in a closely contiguous relationship with the annulus defining the implant site.

It will also be appreciated from observation of Figure 4 that the annular formation 100 preferably does not have a flat but rather a slightly undulating form with crests located in correspondence with the commissures of the valve membranes 94. It is in fact established that such a configuration allows, on one hand, further to reduce the axial dimension of the prosthesis and, on the other hand, to obtain an even better adaptation of the prosthesis to the physiology of the implant site.

A substantially similar support formation, indicated 102, also made of shape memory material such as nitinol, which can also be made in the shape of a continuous or substantially continuous body both by juxtaposition of several arcuate segments, is applied (by stitching) to the output edge (outflow side) of the outer sleeve 90 of the prosthesis.

Both the annular formations 100 and 102 have characteristics of flexibilty (due both to the flexibility of the constitutent material and to their thin section: in fact very thin wire-like elements are used, for example with circular section and a diameter of the order of tenths of mm) which makes it possible to mount the prosthesis on a catheter or other suitable instrument for introduction into the implant.

It will be appreciated however that, although less preferred, the prosthesis 88 could be also provided with only one of the formations 100 and 102.

In any case, the prosthesis 88 lends itself, in an arrangement that has been found to be preferential, to be folded into a generally lobed configuration (for example by forming three lobes the radially outer vertices of which correspond to the commissures of the valve) and then rolled by acting tangentally in a manner entirely similar to that adopted for the folds of the cover of a closed umbrella for folding it around the umbrella frame itself. In these conditions the prostheses 88 is reduced to a cylinder having a length of the order of 1.5-2.5 cm (depending on the overall dimensions of the prostheses, which in turn depends on the type of valve replaced and the anthropometric characteristics of the patient) and a diameter of the order of several millimetres so as to allow its positioning on a suitable instrument for low invasive surgery techniques.

Having reached the implant site the prosthesis 88 can be unfolded by making it re-acquire the functional shape illustrated in Figure 1 by heating to moderate temperature. For example, with reference to the choice of a material such as nitinol, for producing the formations 100 and 102, this unfolding temperature can be greater than that of the body in the operating conditions, and preferably of the order of 50°C so as not to create detrimental effects on the prostheses, the surrounding tissue or the organism of the patient in general.

The heating can easily be caused for example by utilising a transport catheter provided, at its distal end, with an expandable balloon situated in correspondence with the region intended to receive the valve and retain it in position. By introducing hot water through the catheter into the balloon it is possible to induce the recovery of the shape memory metal into the definitive shape.

Alternatively it is possible to effect heating of the support formations 100, 102 by the Joule effect by means of an induced electric current by electromagnetic coupling via a suitable device located in proximity thereof.

In order to anchor the prostheses 88 in situ it is possible to utilise various arrangements, for example that already described with reference to the rings of the preceding Figures 1 to 3, which envisages the use of projections 104 of shape memory material, preferably having different thermal properties from those of the material utilised for the support formations 100 and 102.

In the retracted condition of the prosthesis 88 the projections 104 are collapsed upon themselves in a strongly curved shape, which they also maintain during the heating phase which allows unfolding of the support formations 100, 102.

Subsequently, by the effect of a further heating to a higher temperature than that necessary to cause unfolding of the support formations 100, 102, the projections 104 extend in a hook like form, opening outwardly, and penetrate into the vasal wall or into the implanted valve annulus so as to anchor the prostheses 88 in situ.

Naturally, it is possible to use other anchorage arrangements, for example arrangements such as those currently adopted for anchorage in situ of vascular (graft) prostheses introduced endoluminally. By the way, these other arrangements could be utilised, at least in principle, also for the anchorage of a ring for annuloplasty of the type described with reference to the preceding Figures from 1 to 3. By way of example one may cite the arrangements described in US Patents US-A-4 787 899, US-A-5 104 399, US-A-5 256 150 and US-A-5 275 622 or else by having recourse to an endoluminal stent of the type known as a Palmaz stent stitched to the inlet edge of the valve prosthesis which in this case is made with a slightly longer shape (this arrangement appears to be preferable for use in replacement of the pulmonary valve).

In the case of use for replacement of the aortic valve it can be advantageous to provide the inlet edge of the prosthesis 88 with a strip of biological or polymeric tissue (schematically shown with the broken line and indicated 106 in Figure 4) for the purpose of acting as a seal.

It is also possible to consider duplicating the function of the projections 104 with respect to that of the formation 100 by arranging them on another ring element (not shown in Figure 4) formed with material having a shape memory and possibly disposed in a marginal position with respect to the edge 106.

Again, another arrangement usable for anchoring the prostheses 88 in situ is that based on the use of helices which can be controlled to rotate remotely by means of catheters as described in Italian Patent application BO94A000552.

Again, since it can be critical to facilitate the orientation of the valve by the surgeon during the operation, it is preferable that in correspondence with the commissural supports there be located radio opaque markers connected to the introduction instrument and capable of being removed after positioning of the prosthesis.

Naturally, the principle of the invention remaining the same, the details of construction and the embodiments can be widely varied with respect to what has been described and illustrated, without by this departing from the ambit of the present invention: this relates in particular to the choice of shape-memory material usable for the support formations.

## Claims

1. A device (10, 10a, 88) for cardiac valve replacement or repair operations, comprising at least one support formation (12, 100, 102) of shape-memory material which can adopt:
- a first, generally contracted shape, in which the said formation (12, 100, 102) can be wound on itself or folded for the purpose of location in the implant position, with low-invasive surgical techniques, and
- a second, generally unfolded shape, to which the said shape memory material can be brought after positioning of the support formation (12, 100, 102) in the implant position,
as well as anchorage means for fixing the device in the implant position, which anchorage means comprise formations capable of extending outwardly from the said support formation (12, 100, 102) and which are projections (16, 104) of the said at least one support formation (12, 100, 102),
said device being **characterised in that** the said projections (16, 104) are made of a shape-memory material different from that utilised for the said support formation and which can adopt:
- a first shape, usable upon implant of the device, in which the said projections (16, 104) are generally contracted, and
- a second shape, usable when the device is in the unfolded position, in which the said projections (16, 104) extend outwardly from the device itself, the passage from the said first to the said second shape occurring by the effect of heating to a temperature greater than that at which the passage from one shape to the other of the shape memory material used for the said support formation (12, 100, 102) takes place.

2. A device (10, 10a, 88) according to Claim 1, **characterised in that** the said shape memory material is a metal and the passage from the said first to the said second shape is achieved by heating.

3. A device (10, 10a, 88) according to Claim 2, **characterised in that** the said metal is nitinol.

4. A device (10, 10a, 88) according to Claim 2 or Claim 3, **characterised in that** the said shape memory material passes from the said first to the said second shape by the effect of heating to a temperature of the order of about 50°C.

5. A device (10, 10a, 88) according to any preceding claim, **characterised in that** the said at least one support formation (12, 100, 102) is of generally annular shape.

6. A device (10, 88) according to Claim 5, **characterised in that** the said at least one support formation (12, 100, 102) is formed in the shape of a continuous annular body.

7. A device (10, 10a) according to any preceding claim **characterised in that** it is a ring for annuloplasty.

8. A device (88) according to any of Claims from 1 to 6, , **characterised in that** it is a valve prosthesis comprising a valve sleeve (90, 92) of generally flexible material having respective inlet and outlet edges (99) with respect to the direction of free flow of blood through the prosthesis, the said support formation (100, 102) of shape-memory material being associated with at least one of the said inlet and outlet edges (99).

9. A device (88) according to Claim 8, **characterised in that** there are provided first (100) and second (102) support formations located respectively in correspondence with the said inlet edge and the said outlet edge (99) of the prosthesis.

10. A device (88) according to any of Claims 8 or 9, **characterised in that** the said at least one support formation (100) is located in correspondence with the inlet edge of the prosthesis and has a generally festoon configuration with crests located in correspondence with the commissures of the valve membranes (94) of the prosthesis.

11. A device (88) according to any of Claims from 8 to 10; **characterised in that** the said at least one support formation (102) is located in correspondence with the outlet edge of the prosthesis and has a generally festoon configuration with crests located in correspondence with the commissures of the valve membranes (94) of the prosthesis.

12. A device (88) according to Claim 11, **characterised in that** the said valve sleeve comprises an outer layer (90) and an inner layer (92) carrying the valve membranes (94) of the prosthesis, the said outer layer (90) and the said inner layer (92) being connected together by a line of stitching (96) having a generally festoon configuration and **in that** a respective support formation (102) is located in correspondence with the said outlet edge (99) closely adjacent the said stitch line (96).

13. A device (88) according to any of Claims from 8 to 12, **characterised in that** it comprises, in correspondence with the said inlet edge, a seal strip of sheet material (106) which can be fitted in contact with the wall of the implant site.

## Patentansprüche

1. Vorrichtung (10, 10a, 88) für den Herzklappenersatz oder für Wiederherstellungsoperationen, umfassend zumindest ein Stützgebilde (12, 100, 102) aus Formgedächtnismaterial, welches folgende Formen annehmen kann:
- eine erste, im Allgemeinen kontrahierte Form, bei der das Gebilde (12, 100, 102) zum Zweck der Platzierung an der Implantatposition durch wenig invasive chirurgische Techniken um sich selbst gewunden oder gefaltet werden kann, und
- eine zweite, im Allgemeinen ungefaltete Form, in welche das Formgedächtnismaterial nach der Positionierung des Stützgebildes (12, 100, 102) an der Implantatposition gebracht werden kann,
sowie Verankerungsmittel zum Befestigen der Vorrichtung an der Implantatposition, welche Verankerungsmittel Gebilde umfassen, die in der Lage sind, sich vom Stützgebilde (12, 100, 102) nach außen auszudehnen, und die Vorsprünge (16, 104) des zumindest einen Stützgebildes (12, 100, 102) sind,
wobei die Vorrichtung **dadurch gekennzeichnet ist, dass** die Vorsprünge (16, 104) aus einem Formgedächtnismaterial gefertigt sind, welches sich von jenem unterscheidet, das für das Stützgebilde verwendet wird, und welches folgende Formen annehmen kann:
- eine erste Form, die beim Implantieren der Vorrichtung verwendbar ist und bei der die Vorsprünge (16, 104) im Allgemeinen eingezogen sind, und
- eine zweite Form, die verwendbar ist, wenn sich die Vorrichtung in der ungefalteten Stellung befindet, und bei der sich die Vorsprünge (16, 104) von der Vorrichtung selbst nach außen ausdehnen, wobei der Übergang von der ersten zur zweiten Form durch die Wirkung einer Erhitzung auf eine Temperatur erfolgt, die höher ist als jene, bei welcher der Übergang von einer Form zur anderen des für das Stützgebilde (12, 100, 102) verwendeten Formgedächtnismaterials stattfindet.

2. Vorrichtung (10, 10a, 88) gemäß Anspruch 1, **dadurch gekennzeichnet, dass** das Formgedächtnismaterial ein Metall ist und der Übergang von der ersten zur zweiten Form durch Erhitzung erzielt wird.

3. Vorrichtung (10, 10a, 88) gemäß Anspruch 2, **dadurch gekennzeichnet, dass** das Metall Nitinol ist.

4. Vorrichtung (10, 10a, 88) gemäß Anspruch 2 oder Anspruch 3, **dadurch gekennzeichnet, dass** das Formgedächtnismaterial durch die Wirkung einer Erhitzung auf eine Temperatur in der Größenordnung von etwa 50°C von der ersten zur zweiten Form übergeht.

5. Vorrichtung (10, 10a, 88) gemäß irgendeinem vorhergehenden Anspruch, **dadurch gekennzeichnet, dass** das zumindest eine Stützgebilde (12, 100, 102) im Allgemeinen eine Ringform aufweist.

6. Vorrichtung (10, 88) gemäß Anspruch 5, **dadurch gekennzeichnet, dass** das zumindest eine Stützgebilde (12, 100, 102) in Form eines durchgehenden ringförmigen Körpers gestaltet ist.

7. Vorrichtung (10, 10a) gemäß irgendeinem vorhergehenden Anspruch, **dadurch gekennzeichnet, dass** sie ein Ring für die Annuloplastie ist.

8. Vorrichtung (88) gemäß einem der Ansprüche 1 bis 6, **dadurch gekennzeichnet, dass** sie eine Klappenprothese ist, umfassend eine Klappenhülse (90, 92) aus einem im Allgemeinen biegsamen Material, welche entsprechende Eingangs- und Ausgangsränder (99) bezüglich der Richtung des freien Blutflusses durch die Prothese aufweist, wobei das Stützgebilde (100, 102) aus Formgedächtnismaterial mit zumindest einem der Eingangs- und Ausgangsränder (99) verbunden ist.

9. Vorrichtung (88) gemäß Anspruch 8, **dadurch gekennzeichnet, dass** erste (100) und zweite (102) Stützgebilde vorgesehen sind, die jeweils in Übereinstimmung mit dem Eingangsrand und dem Ausgangsrand (99) der Prothese positioniert sind.

10. Vorrichtung (88) gemäß einem der Ansprüche 8 oder 9, **dadurch gekennzeichnet, dass** das zumindest eine Stützgebilde (100) in Übereinstimmung mit dem Eingangsrand der Prothese positioniert ist und eine im Allgemeinen girlandenförmige Struktur aufweist, wobei die Scheitelpunkte in Übereinstimmung mit den Kommissuren der Klappenmembranen (94) der Prothese positioniert sind.

11. Vorrichtung (88) gemäß einem der Ansprüche 8 bis 10, **dadurch gekennzeichnet, dass** das zumindest eine Stützgebilde (102) in Übereinstimmung mit dem Ausgangsrand der Prothese positioniert ist und eine im Allgemeinen girlandenförmige Struktur aufweist, wobei die Scheitelpunkte in Übereinstimmung mit den Kommissuren der Klappenmembranen (94) der Prothese positioniert sind.

12. Vorrichtung (88) gemäß Anspruch 11, **dadurch gekennzeichnet, dass** die Klappenhülse eine Außenschicht (90) und eine die Klappenmembranen (94) der Prothese tragende Innenschicht (92) umfasst, wobei die Außenschicht (90) und die Innenschicht (92) durch eine Nahtlinie (96) mit im Allgemeinen girlandenförmiger Struktur miteinander verbunden sind, und dass ein entsprechendes Stützgebilde (102) in Übereinstimmung mit dem Ausgangsrand (99) nahe angrenzend an die Nahtlinie (96) positioniert ist.

13. Vorrichtung (88) gemäß einem der Ansprüche 8 bis 12, **dadurch gekennzeichnet, dass** sie in Übereinstimmung mit dem Eingangsrand einen Dichtungsstreifen aus Flächenmaterial (106) umfasst, welcher in Kontakt mit der Wand der Implantatstelle eingesetzt werden kann.

## Revendications

1. Dispositif (10, 10a, 88) pour des opérations de remplacement ou de réparation de valves cardiaques, comprenant au moins une formation d'appui (12, 100, 102) constituée d'une matière à mémoire de forme qui peut adopter :
- une première forme globalement contractée, dans laquelle ladite formation (12, 100, 102) peut être enroulée sur elle-même ou pliée aux fins de positionnement dans la position d'implantation avec des techniques chirurgicales peu invasives, et
- une seconde forme globalement dépliée, vers laquelle ladite matière à mémoire de forme peut être amenée après le positionnement de la formation d'appui (12, 100, 102) dans la position d'implantation,
ainsi que des moyens d'ancrage pour fixer le dispositif dans la position d'implantation, lesquels moyens d'ancrage comprennent des formations capables de s'étendre vers l'extérieur depuis ladite formation d'appui (12, 100, 102) et qui sont des saillies (16, 104) de ladite au moins une formation d'appui (12, 100, 102),
ledit dispositif étant **caractérisé en ce que** lesdites saillies (16, 104) sont constituées d'une matière à mémoire de forme différente de celle utilisée pour ladite formation d'appui et qui peut adopter :
- une première forme, utilisable lors de l'implantation du dispositif, dans laquelle lesdites saillies (16, 104) sont globalement contractées, et
- une seconde forme, utilisable lorsque le dispositif est dans la position dépliée, dans laquelle lesdites saillies (16, 104) s'étendent vers l'extérieur depuis le dispositif lui-même, le passage de ladite première forme à ladite seconde forme se produisant par l'effet de chauffage à une température supérieure à celle à laquelle le passage d'une forme à l'autre de la matière à mémoire de forme utilisée pour ladite formation d'appui (12, 100, 102) se produit.

2. Dispositif (10, 10a, 88) selon la revendication 1, **caractérisé en ce que** ladite matière à mémoire de forme est un métal et le passage de ladite première forme à ladite seconde forme est réalisé par chauffage.

3. Dispositif (10, 10a, 88) selon la revendication 2, **caractérisé en ce que** ledit métal est le nitinol.

4. Dispositif (10, 10a, 88) selon la revendication 2 ou la revendication 3, **caractérisé en ce que** ladite matière à mémoire de forme passe de ladite première forme à ladite seconde forme par l'effet de chauffage à une température de l'ordre d'environ 50°C.

5. Dispositif (10, 10a, 88) selon l'une quelconque des revendications précédentes, **caractérisé en ce que** ladite au moins une formation d'appui (12, 100, 102) est d'une forme globalement annulaire.

6. Dispositif (10, 88) selon la revendication 5, **caractérisé en ce que** ladite au moins une formation d'appui (12, 100, 102) est réalisée sous la forme d'un corps annulaire continu.

7. Dispositif (10, 10a) selon l'une quelconque des revendications précédentes, **caractérisé en ce qu'**il s'agit d'un anneau pour annuloplastie.

8. Dispositif (88) selon l'une quelconque des revendications 1 à 6, **caractérisé en ce qu'**il s'agit d'une prothèse de valve comprenant un manchon de valve (90, 92) constitué d'une matière globalement flexible comportant des arêtes d'entrée et de sortie respectives (99) par rapport à la direction d'écoulement libre du sang à travers la prothèse, ladite formation d'appui (100, 102) constituée d'une matière à mémoire de forme étant associée à au moins l'une desdites arêtes d'entrée et de sortie (99).

9. Dispositif (88) selon la revendication 8, **caractérisé en ce que** des première (100) et seconde (102) formations d'appui sont prévues de manière à être placées respectivement en correspondance avec ladite arête d'entrée et ladite arête de sortie (99) de la prothèse.

10. Dispositif (88) selon l'une quelconque des revendications 8 ou 9, **caractérisé en ce que** ladite au moins une formation d'appui (100) est située en correspondance avec l'arête d'entrée de la prothèse et a une configuration globalement en feston avec des crêtes situées en correspondance avec les commissures des membranes de valve (94) de la prothèse.

11. Dispositif (88) selon l'une quelconque des revendications 8 à 10, **caractérisé en ce que** ladite au moins une formation d'appui (102) est située en correspondance avec l'arête de sortie de la prothèse et a une configuration globalement en feston avec des crêtes situées en correspondance avec les commissures des membranes de valve (94) de la prothèse.

12. Dispositif (88) selon la revendication 11, **caractérisé en ce que** ledit manchon de valve comprend une couche extérieure (90) et une couche intérieure (92) portant les membranes de valve (94) de la prothèse, ladite couche extérieure (90) et ladite couche intérieure (92) étant reliées ensemble par une ligne de piqûre (96) ayant une configuration globalement en feston et **en ce qu'**une formation d'appui (102) respective est située en correspondance avec ladite arête de sortie (99) très adjacente à ladite ligne de piqûre (96).

13. Dispositif (88) selon l'une quelconque des revendications 8 à 12, **caractérisé en ce qu'**il comprend, en correspondance avec ladite arête d'entrée, une bande de scellement de matière en feuille (106) qui peut être mise en place en contact avec la paroi du site d'implantation.
